# EUROPÄISCHE PATENTSCHRIFT

(11) **EP 3 728 184 B1**
(45) Veröffentlichungstag und Bekanntmachung des Hinweises auf die Patenterteilung: **10.11.2021**
(21) Anmeldenummer: 18819111.8
(22) Anmeldetag: 17.12.2018
(51) Int. Cl.: C07C 267/00

(54) **VERFAHREN ZUR HERSTELLUNG VON CARBODIIMIDEN**
PROCESS FOR THE PREPARATION OF CARBODIIMIDES
PROCEDE DE PREPARATION DE CARBODIIMIDES

(30) Priorität: 20.12.2017 EP 17209011; 28.12.2017 EP 17210799
(43) Veröffentlichungstag der Anmeldung: 28.10.2020
(73) Patentinhaber: LANXESS Deutschland GmbH, 50569 Köln (DE)
(72) Erfinder: LAUFER, Wilhelm, 67158 Ellerstadt (DE)
(86) Internationale Anmeldenummer: PCT/EP2018/085127
(87) Internationale Veröffentlichungsnummer: WO 2019/121462

(56) Entgegenhaltungen:
- EP-A1- 0 602 477

## Beschreibung

Die Erfindung betrifft ein neues Verfahren zur Herstellung von Carbodiimiden, vorzugsweise monomeren, sterisch gehinderten aromatischen Carbodimiden. Carbodiimide haben sich in vielen Anwendungen bewährt, z.B. als Hydrolyseschutzmittel für thermoplastische Kunststoffe, Polyole, Polyurethane, Triglyceride und Schmierstofföle etc.

Die Synthese der Carbodiimide erfolgt nach dem Stand der Technik ausgehend von Isocyanaten, die durch basische oder heterocyclische Katalyse unter CO₂-Abspaltung carbodiimidisiert werden. Dabei können mono- oder polyfunktionelle Isocyanate zu monomeren oder polymeren Carbodiimiden umgesetzt werden.

Die üblicherweise verwendeten Katalysatoren sind Alkali- oder Erdalkaliverbindungen, sowie heterocyclische Verbindungen, die Phosphor enthalten, siehe Angew. Chem. 1962, 74, 801-806 und Angew. Chem. 1981, 93, 855-866.

Schwierig ist die vollständige Entfernung des in der Regel verwendeten Phosphor-haltigen Katalysators. Da Carbodiimide vorzugsweise bei der Herstellung von Polyurethanen eingesetzt werden, ist die Anwesenheit von Phosphor, selbst im Spurenbereich, extrem störend und ist daher zu vermeiden. Darüber hinaus sollten die hergestellten monomeren Carbodiimide eine möglichst geringe Farbzahl und einen geringen Gehalt an Isocyanaten aufweisen, was technisch in der Regel nur durch Destillationen oder mehrmalige Kristallisationen unter Ausbeutenverlusten oder aufwändig durch Umsetzung in Gegenwart von CO₂ zu erreichen ist, siehe EP-A 0 602 477 und EP-A 2 855 573.

Aufgabe der vorliegenden Erfindung war es daher, ein verbessertes Verfahren zur Herstellung bestimmter monomerer Carbodiimide bereitzustellen, welches deren Herstellung mit geringer Farbzahl ermöglich, wobei diese monomeren Carbodiimide idealerweise frei von organischen Phosphorverbindungen und unumgesetzten Ausgangsprodukten, vorzugsweise Isocyanaten, sein sollen, so dass diese bei der Herstellung und/oder Stabilisierung von PU-Systemen eingesetzt werden können.

Überraschenderweise wurde nun gefunden, dass diese vorgenannten Aufgaben erfüllt werden, wenn das Verfahren in folgenden Schritten durchgeführt wird:
- Carbodiimisierung von monomeren aromatischen Isocyanaten in Gegenwart eines Katalysators,
- Abtrennung der Leichtsieder und des Katalysators vom Reaktionsprodukt in einem Dünnschichtverdampfer, und
- Destillation des Rückstandes in einem weiteren Dünnschichtverdampfer.

Gegenstand der vorliegenden Erfindung ist somit ein Verfahren zur Herstellung von monomeren aromatischen Carbodiimiden, welches in folgenden Schritten durchgeführt wird:
- Carbodiimisierung von monomeren aromatischen Isocyanaten in Gegenwart eines Katalysators,
- Abtrennung der Leichtsieder und des Katalysators vom Reaktionsprodukt in einem Dünnschichtverdampfer, und
- Destillation des Rückstandes in einem weiteren Dünnschichtverdampfer.

Monomere aromatische Carbodiimide im Sinne der Erfindung sind dabei Carbodiimide, die C₁₋C₆-alky- und/oder C₁-C₆-alkoxy-substituierte Phenylreste tragen, die direkt über ein Kohlenstoffatom des Phenylrestes oder über eine Alkylgruppe am Phenylrest an den Stickstoff der Carbodiimidgruppe gebunden sind.

Besonders bevorzugt dabei sind monomeren Carbodiimiden der Formel (I)

R ― N = C = N ― R¹ (I)

wobei R und auch R¹ 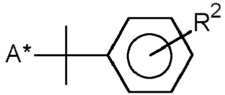 und/oder 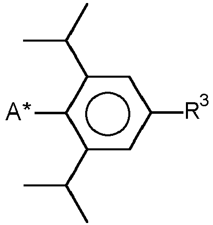 bedeutet
R² = iso-Propenyl, t-Butyl oder -C₁-C₆-Alkoxy ist,
R³ = H, Methyl, Ethyl, iso-Propyl, n-Propyl und/oder tert.-Butyl, vorzugsweise Methyl, Ethyl, iso-Propyl, n-Propyl und/oder tert.-Butyl, ist,
und A* die Verbindung zum Stickstoff der Carbodiimidfunktion in Formel (I) darstellt.

Im Fall der bevorzugten Carbodiimide der Formel (I) erfolgt das erfindungsgemäße Verfahren durch
a) Umsetzung (Carbodiimisierung) von Isocyanaten ausgewählt aus der Gruppe R ― N=C=O und/oder R¹―N=C=O, wobei R und R¹ die vorgenannte Bedeutung haben, in Gegenwart eines Katalysators,
b) Abtrennung der Leichtsieder und des Katalysators vom Reaktionsprodukt aus a) in einem Dünnschichtverdampfer und
c) Destillation des Rückstandes aus b) in einem weiteren Dünnschichtverdampfer.

In einer bevorzugten Ausführungsform des vorliegenden Verfahrens entspricht das Carbodiimid einer Verbindung der Formel (II)

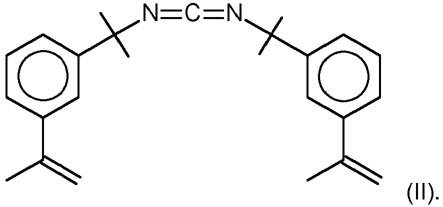

In einer weiteren bevorzugten Ausführungsform des erfindungsgemäßen Verfahrens entspricht das Carbodiimid einer Verbindung der Formel (I) R ― N = C = N ― R¹,
wobei R und R¹ 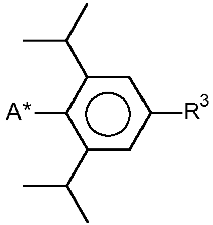 ist, R³ einem iso-Propylrest entspricht und A* die Verbindung zum Stickstoff der Carbodiimidfunktion in Formel (I) darstellt.

Die Carbodiimisierung von Isocyanaten in Gegenwart eines Katalysators im Schritt a) im Sinne des erfindungsgemäßen Verfahrens erfolgt in einer Kondensationsreaktion unter Abspaltung von CO₂, wie beispielsweise beschrieben in der in Angew. Chem. 93, S. 855 - 866 (1981) oder DE-A-11 30 594 oder Tetrahedron Letters 48 (2007), S. 6002 - 6004.

Die Carbodiimidisierung kann sowohl in Substanz als auch in einem Lösemittel durchgeführt werden. Ebenfalls möglich ist es, die Carbodiimidisierung zuerst in Substanz zu starten und anschließend nach Zugabe eines Lösemittels zu vervollständigen. Als Lösemittel werden vorzugsweise Benzine, Benzol und/oder Alkylbenzole eingesetzt.

Als Isocyanate sind vorzugsweise einsetzbar 3-Isopropenyl-α,α-dimethylbenzylisocyanat (TMI) und/oder 2,4,6 Triisopropylphenylisocyanat (TRIPI).

In einer Ausführungsform der Erfindung sind als Katalysatoren für die Herstellung der Verbindungen der Formel (I) Phosphorverbindungen bevorzugt. Vorzugsweise werden Phospholenoxide, Phospholidine oder Phospholinoxide sowie die entsprechenden Sulfide verwendet. Als Katalysator sind besonders Alkylphospholenoxid mit Alkyl = C₁-C₆-Alkyl, vorzugsweise Methylphospholenoxid bevorzugt.

Die Reaktion wird vorzugsweise in einem Temperaturbereich von 40°C bis 200°C durchgeführt.

In einer weiteren bevorzugten Ausführungsform des erfindungsgemäßen Verfahrens findet im Anschluss an die Carbodiimidisierung eine Filtration des Reaktionsproduktes aus a) statt. In diesem Fall erfolgt die Filtration vorzugsweise über Kerzen- oder Beutelfilter gegebenenfalls unter Zugabe von Filtrationshilfsmitteln, wie vorzugsweise Kieselgur oder Aktivkohle.

Vorzugsweise wird die Filtration bei Temperaturen von 30 bis 100°C durchgeführt.

Im Anschluss an die Carbodiimisierung a) und die gegebenenfalls erfolgte Filtration erfolgt die Abtrennung der Leichtsieder und des Katalysators vom Reaktionsprodukt aus a) in einem Dünnschichtverdampfer.

Der Dünnschichtverdampfer im Sinne der Erfindung ist ein Verdampfer, der Flüssigkeiten in einem dünnen Film verdampft. Er wird vorzugsweise im Vakuum betrieben und eignet sich deshalb zum schonenden Trennen von Stoffgemischen. Unter Dünnschichtverdampfer im Sinne der Erfindung sind Fallfilmverdampfer mit rotierenden Wischern bevorzugt, beispielsweise erhältlich bei der Firma VTA GmbH & Co. KG.

Leichtsieder in Sinne der Erfindung sind vorzugsweise die während der Durchführung der Carbodiimidisierung entstehenden Nebenprodukte oder nicht umgesetzte Edukte/Isocyanate mit Siedepunkten, die mindestens 50°C, bevorzugt mindestens 60°C besonders bevorzugt mindestens 70°C unter denen des zuvor hergestellten monomeren Carbodiimides oder des entsprechenden Carbodiimidgemisches liegen.

Die Abtrennung im Schritt b) erfolgt vorzugsweise bei einer Temperatur von 150 °C bis 200 °C, bevorzugt 160 °C bis 190 °C, besonders bevorzugt 170 °C bis 180 °C und einem Druck von 0,1 bis 5 mbar, bevorzugt 0,2 - 1 mbar, besonders bevorzugt 0,3 - 0,6 mbar.

In einer weiteren bevorzugten Ausführungsform der Erfindung beträgt die Temperaturdifferenz zwischen Schritt b) und Schritt c) 5°C, vorzugsweise 10°C.

In einer weiteren bevorzugten Ausführungsform der Erfindung liegt die Temperatur in Schritt c) um 5°C, vorzugsweise 10°C höher als in Schritt b).

In einer weiteren Ausführungsform können sogenannte Schleppmittel bei der Entfernung der Leichtsieder eingesetzt werden. Bevorzugt sind hier C₁―C₁₂- alkylsubstituierte Benzole und/oder Dibenzole und/oder Pyrrolidone. Hierbei bevorzugt sind N-Methyl-, N-Ethylpyrrolidon und/oder Xylol.

Im Schritt c) erfolgt die Destillation des Rückstandes aus b) in einem weiteren Dünnschichtverdampfer. Bevorzugt erfolgt die Destillation des Rückstandes aus c) in einem weiteren Dünnschichtverdampfer. Als Dünnschichtverdampfer wird vorzugsweise ein Kurzwegverdampfer eingesetzt.

Kurzwegverdampfer im Sinne der Erfindung sind modifizierte Dünnschichtverdampfer, bei denen der Kondensator in das Innere des Verdampferzylinders integriert ist. Dadurch ist der Weg, den die Dämpfe vom Produktfilm zum Kondensator zurücklegen müssen, sehr kurz.

Der Rückstand aus b) im Sinne der Erfindung beinhaltet dabei vor allem die zuvor hergestellten monomeren Carbodiimide und daraus resultierende Nebenprodukte.

Im Schritt c) erfolgt die Destillation des Rückstandes aus b) vorzugsweise bei einer Temperatur von 160°C bis 220°C, bevorzugt 165°C bis 210°C, besonders bevorzugt 190°C bis 205°C und einem Druck von 0,05 bis 5 mbar, bevorzugt 0,1 - 1 mbar, besonders bevorzugt 0,2 - 0,6 mbar.

In einer weiteren bevorzugten Ausführungsform der Erfindung werden die Schritte b) und c) ohne Isolierung des Rückstandes aus b) kontinuierlich durchgeführt.

Die nach dem erfindungsgemäßen Verfahren hergestellten monomeren Carbodiimide weisen vorzugsweise eine Farbzahl von < 10 im b-Wert, gemessen nach CIE L*a*b* nach ISO 11664-4 in Kombination mit geringen Anteilen an monomerem Isocyanat von < 0,1% und Phosphorgehalt < 1 ppm auf.

Ein weiterer Gegenstand der vorliegenden Erfindung ist die Verwendung der nach dem erfindungsgemäßen Verfahren hergestellten monomeren Carbodiimide zur Herstellung von hydrolysestabilen Polyurethan(PU)-basierten Systemen, vorzugsweise thermoplastisches Polyurethan (TPU), PU-Weich- und Hartschaum sowie PU-Heißgießelastomere.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Ausführungsbeispiele:

CDI 1: 2,6-Diisopropylphenylcarbodiimid
CDI 2: Carbodiimid der Formel (II) 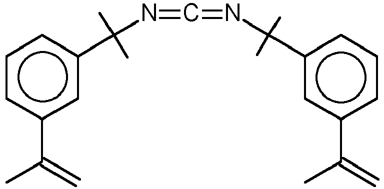
CDI 3: Carbodiimid der Formel (I) R ― N = C = N ― R¹ , wobei R und R¹ 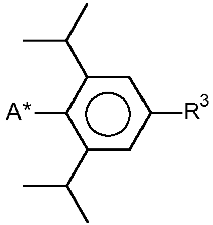 bedeuten und R³ iso-Propyl entspricht und wobei A* die Verbindung zum Stickstoff der Carbodiimidfunktion in Formel (I) darstellt.

### Carbodiimidisierung

Die Herstellung der Carbodiimide CDI 1 bis 3 erfolgte in einem Edelstahlkessel durch die Umsetzung der entsprechenden Isocyanate, d.h. für CDI 1 wurde 2,6-Diisopropylphenylisocyanat (DIPPI), für CDI 2 wurde 3-Isopropenyl-α,α-dimethylbenzyl-isocyanat (TMI) und für CDI 3 wurde 2,4,6 Triisopropylphenylisocyanat (TRIPI) eingesetzt, in Gegenwart von 200 - 500 ppm Methylphospholenoxid als Katalysator bei Temperaturen von 160 - 170°C unter Abspaltung von CO₂. Es wurde so lange carbodiimidisiert bis ein Restisocyanat-Gehalt von < 1 % erreicht wurde.

Die so hergestellten Carbodiimide CDI 1 bis CDI 3 wurden anschließend den nachstehend aufgeführten Destillationen zugeführt.

### a) Destillation und Destillation im Dünnschichtverdampfer (Vergleich) gemäß EP-A-0 602 477:

Die Carbodiimide CDI 1 bis 3 wurden zuerst im Batch von den Leichtsiedern bei ca. 200 - 220 °C und einem Druck 0,3 - 0,4 mbar über eine Vigreux-Kolonne abgetrennt und anschließend in einem Dünnschichtverdampfer der Firma VTA bei Temperaturen 190 - 200°C und einem Druck von 0,4 mbar kontinuierlich abdestilliert.

### b) Destillation über 2 Dünnschichtverdampfer (erfindungsgemäß):

Die Carbodiimide CDI 1 bis 3 wurden zuerst in einem Dünnschichtverdampfer der Firma VTA bei Temperaturen von 160 - 170 °C bei einem Druck von 1,0 mbar von Leichtsiedern befreit und der Rückstand in einem zweiten, zu dem ersten identischen Dünnschichtverdampfer bei Temperaturen von 200 - 205°C und einem Druck von 0,4 mbar abdestilliert.

Die Farbbestimmung wurde nach der CIE L*a*b* Methode gemäß ISO 11664-4 durchgeführt. Bewertet wurde der b*-Wert.

Der Restgehalt an monomerem Isocyanat wurde mittels HPLC und der Phosphor-Gehalt mittels Röntgen-Fluoreszenz-Analyse (RFA) bestimmt.

Die Ergebnisse sind in Tabelle 1 nachstehend aufgeführt.

**Tabelle 1:**

| **CDI** | **Destillation + Destillation im Dünnschichtverdampfer (Vgl.)** | | | **2 Dünnschichtverdampfer (Erf.)** | | |
|---|---|---|---|---|---|---|
| | Farbe, **b**^{∗} | Gehalt an monomerem Isocyanat (%) | Phosphor-Gehalt (ppm) | Farbe, **b**^{∗} | Gehalt an monomerem Isocyanat (%) | Phosphor-Gehalt (ppm) |
| **CDI 1** | **10-12** | **> 0,1** | **4-5** | **2-3** | **> 0,1** | **3** |
| **CDI 2** | **5-6** | **> 0,1** | **10-12** | **0-1** | **< 0,1** | **< 1**^{∗∗} |
| **CDI 3** | **30-40** | **> 0,1** | **5-6** | **8-9** | **< 0,1** | **< 1**^{∗∗} |

| | | | | | | |
|---|---|---|---|---|---|---|
| Vgl. = Vergleich Erf. = erfindungsgemäß ^{∗∗} Unter der Nachweisgrenze von 1 ppm | | | | | | |

Die nach dem erfindungsgemäßen Verfahren hergestellten Carbodiimide CDI 1 bis CDI 3 weisen gegenüber denen, die nach dem Verfahren gemäß Stand der Technik hergestellt wurden, verbesserte Eigenschaften auf.

Wie aus Tabelle 1 ersichtlich, können insbesondere die bevorzugten Carbodiimide CDI 2 und CDI 3 in besonders hoher Qualität, d.h. geringer Farbzahl, niedrigem Gehalt an giftigem monomeren Isocyanat hergestellt werden und weisen zudem keinen nachweisbaren Gehalt an organischen Phosphorverbindungen auf.

Diese eignen sich in idealer Weise für ihren Einsatz bei der Herstellung und/oder Stabilisierung von PU-Systemen.

## Patentansprüche

1. Verfahren zur Herstellung von monomeren aromatischen Carbodiimiden
**dadurch gekennzeichnet, dass** dies in folgenden Schritten durchgeführt wird
a) Carbodiimisierung von monomeren aromatischen Isocyanaten in Gegenwart eines Katalysators
b) Abtrennung der Leichtsieder und des Katalysators vom Reaktionsprodukt aus a) in einem Dünnschichtverdampfer,
c) Destillation des Rückstandes aus b) in einem weiteren Dünnschichtverdampfer.

2. Verfahren nach Anspruch 1, **dadurch gekennzeichnet, dass** es sich bei den monomeren aromatischen Carbodiimiden um Carbodiimide der Formel (I)
R ― N = C = N ― R¹ (I)
handelt
wobei R und R¹ 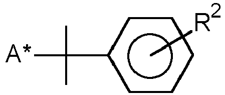 und/oder 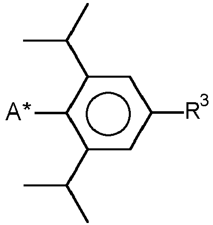 bedeutet
R² = iso-Propenyl, t-Butyl oder C₁-C₆-Alkoxy,
R³ = H, Methyl, Ethyl, iso-Propyl, n-Propyl und/oder tert.-Butyl, vorzugsweise Methyl, Ethyl, iso-Propyl, n-Propyl und/oder tert.-Butyl ist, und
A* die Verbindung zum Stickstoff der Carbodiimidfunktion in Formel (I) darstellt.

3. Verfahren nach Anspruch 1 oder 2, **dadurch gekennzeichnet, dass** das Carbodiimid der Formel (I) 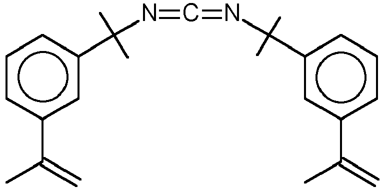 entspricht.

4. Verfahren nach einem der Ansprüche 1 bis 3, **dadurch gekennzeichnet, dass** die Destillationsschritte b) und c) kontinuierlich durchgeführt werden.

5. Verfahren nach einem der Ansprüche 1 bis 4, **dadurch gekennzeichnet, dass** die Destillation in Schritt b) bei einer Temperatur von 150 °C bis 220°C, bevorzugt 160°C bis 200°C, besonders bevorzugt 170°C bis 190°C und einem Druck von 0,1 bis 5 mbar, bevorzugt 0,2 - 1 mbar, besonders bevorzugt 0,3 - 0,6 mbar durchgeführt wird.

6. Verfahren nach einem der Ansprüche 1 bis 5, **dadurch gekennzeichnet, dass** bei der Entfernung der Leichtsieder als Schleppmittel C₁-C₁₂-alkylsubstituierte Benzole und/oder Dibenzole und/oder Pyrrolidone eingesetzt werden.

7. Verfahren nach einem der Ansprüche 1 bis 6, **dadurch gekennzeichnet, dass** die Temperatur in Schritt c) 5°C, vorzugsweise 10°C höher ist als in Schritt b).

8. Verfahren nach einem der Ansprüche 1 bis 6, **dadurch gekennzeichnet, dass** die Destillation in Schritt Im Schritt c) bei einer Temperatur von 160°C bis 220°C, bevorzugt 165°C bis 210°C, besonders bevorzugt 180°C bis 205°C und einem Druck von 0,05 bis 5 mbar, bevorzugt 0,1 - 1 mbar, besonders bevorzugt 0,2 - 0,6 mbar durchgeführt wird.

9. Verfahren nach einem der Ansprüche 1 bis 8, **dadurch gekennzeichnet, dass** als Isocyanate 3-Isopropenyl-α,α-dimethylbenzyl-isocyanat (TMI) und/oder 2,4,6-Triisopropylphenylisocyanat (TRIPI) eingesetzt werden.

10. Verfahren nach einem der Ansprüche 1 bis 9, **dadurch gekennzeichnet, dass** als Dünnschichtverdampfer in Schritt c) ein Kurzwegverdampfer eingesetzt wird.

11. Verfahren nach einem der Ansprüche 1 bis 10, **dadurch gekennzeichnet, dass** die darüber hergestellten Carbodiimide der Formel (I) zur Herstellung von hydrolysestabilen Polyurethan(PU)-basierten Systemen, vorzugsweise thermoplastisches Polyurethan (TPU), PU-Weich- und Hartschaum sowie PU-Heißgießelastomere, eingesetzt werden.

## Claims

1. Method for preparing monomeric aromatic carbodiimides
**characterized in that** this is carried out in the following steps
a) carbodiimidizing monomeric aromatic isocyanates in the presence of a catalyst,
b) separating the low boilers and catalyst from the reaction product from a) in a thin-film evaporator,
c) distilling the residue from b) in a further thin-film evaporator.

2. Method according to Claim 1, **characterized in that** the monomeric aromatic carbodiimides are carbodiimides of the formula (I)
R―N=C=N―R¹ (I),
where R and R¹ are 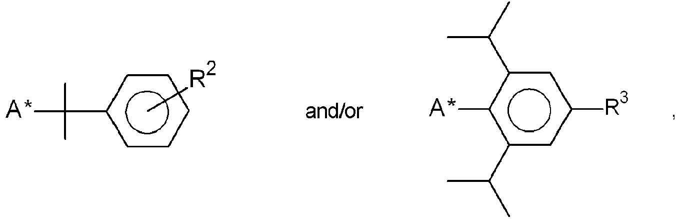
R² = isopropenyl, t-butyl or C₁-C₆ alkoxy,
R³ = H, methyl, ethyl, isopropyl, n-propyl and/or tert-butyl, preferably methyl, ethyl, isopropyl, n-propyl and/or tert-butyl, and
A* represents the linkage to the nitrogen of the carbodiimide function in formula (I).

3. Method according to Claim 1 or 2, **characterized in that** the carbodiimide of the formula (I) corresponds to

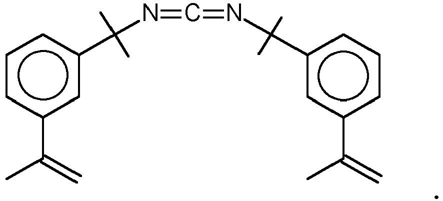

4. Method according to any of Claims 1 to 3, **characterized in that** the distillation steps b) and c) are operated as a continuous process.

5. Method according to any of Claims 1 to 4, **characterized in that** the distillation in step b) is carried out at a temperature of 150°C to 220°C, preferably 160°C to 200°C, more preferably 170°C to 190°C, and at a pressure of 0.1 to 5 mbar, preferably 0.2-1 mbar, more preferably 0.3-0.6 mbar.

6. Method according to any of Claims 1 to 5, **characterized in that** C₁-C₁₂ alkyl-substituted benzenes and/or dibenzenes and/or pyrrolidones are used as entraining agents in the removal of low boilers.

7. Method according to any of Claims 1 to 6, **characterized in that** the temperature in step c) is 5°C higher, preferably 10°C higher, than in step b) .

8. Method according to any of Claims 1 to 6, **characterized in that** the distillation in step in step c) is carried out at a temperature of 160°C to 220°C, preferably 165°C to 210°C, more preferably 180°C to 205°C, and at a pressure of 0.05 to 5 mbar, preferably 0.1-1 mbar, more preferably 0.2-0.6 mbar.

9. Method according to any of Claims 1 to 8, **characterized in that** the isocyanate used is 3-isopropenyl-a,a-dimethylbenzyl isocyanate (TMI) and/or 2,4,6-triisopropylphenyl isocyanate (TRIPI).

10. Method according to any of Claims 1 to 9, **characterized in that** the thin-film evaporator used in step c) is a short-path evaporator.

11. Method according to any of Claims 1 to 10, **characterized in that** the carbodiimides of the formula (I) thereby prepared are used in the production of hydrolysis-stable polyurethane-based (PU-based) systems, preferably thermoplastic polyurethane (TPU), soft and rigid PU foam, and hot-cast PU elastomers.

## Revendications

1. Procédé de préparation de carbodiimides aromatiques monomères, **caractérisé en ce qu'**il est mis en œuvre dans les étapes suivantes :
a) carbodiimidation d'isocyanates aromatiques monomères en présence d'un catalyseur,
b) séparation des substances à bas point d'ébullition et du catalyseur d'avec le produit de réaction de a) dans un évaporateur à couche mince,
c) distillation du résidu de b) dans un autre évaporateur à couche mince.

2. Procédé selon la revendication 1, **caractérisé en ce que**, pour ce qui concerne les carbodiimides aromatiques monomères, il s'agit de carbodiimides de formule (I)
R―N=C=N―R¹ (I)
dans laquelle R et R¹ représentent 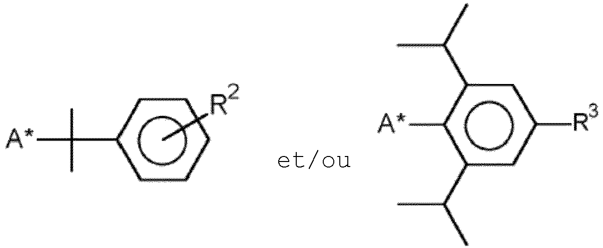
R² = isopropényle, t-butyle ou alcoxy en C₁-C₆,
R³ = H, méthyle, éthyle, isopropyle, n-propyle et/ou tert.-butyle, de préférence méthyle, éthyle, isopropyle, n-propyle et/ou tert.-butyle, et
A* représente la liaison à l'azote de la fonction carbodiimide dans la formule (I).

3. Procédé selon la revendication 1 ou 2, **caractérisé en ce que** le carbodiimide correspond à la formule (I)

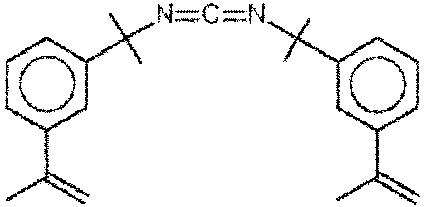

4. Procédé selon l'une des revendications 1 à 3, **caractérisé en ce que** les étapes de distillation b) et c) sont mises en œuvre en continu.

5. Procédé selon l'une des revendications 1 à 4, **caractérisé en ce que** la distillation dans l'étape b) est mise en œuvre à une température de 150 °C à 220 °C, de préférence de 160 °C à 200 °C, d'une manière particulièrement préférée de 170 °C à 190 °C et sous une pression de 0,1 à 5 mbar, de préférence de 0,2 à 1 mbar, d'une manière particulièrement préférée de 0,3 à 0,6 mbar.

6. Procédé selon l'une des revendications 1 à 5, **caractérisé en ce que**, lors de l'élimination des substances à bas point d'ébullition, on utilise comme entraîneur des benzènes et/ou des dibenzènes et/ou des pyrrolidones à substitution alkyle en C₁-C₁₂.

7. Procédé selon l'une des revendications 1 à 6, **caractérisé en ce que** la température dans l'étape c) est de 5 °C, de préférence de 10 °C supérieure à la température dans l'étape b).

8. Procédé selon l'une des revendications 1 à 6, **caractérisé en ce que** la distillation dans l'étape c) est mise en œuvre à une température de 160 °C à 220 °C, de préférence de 165 °C à 210 °C, d'une manière particulièrement préférée de 180 °C à 205 °C et sous une pression de 0,05 à 5 mbar, de préférence de 0,1 à 1 mbar, d'une manière particulièrement préférée de 0,2 à 0,6 mbar.

9. Procédé selon l'une des revendications 1 à 8, **caractérisé en ce qu'**on utilise en tant qu'isocyanates de l'isocyanate de 3-isopropényl-α,α-diméthylbenzyle (TMI) et/ou de l'isocyanate de 2,4,6-triisopropylphényle (TRIPI).

10. Procédé selon l'une des revendications 1 à 9, **caractérisé en ce qu'**on utilise comme évaporateur à couche mince dans l'étape c) un évaporateur direct.

11. Procédé selon l'une des revendications 1 à 10, **caractérisé en ce que** les carbodiimides de formule (I) préparés par ce procédé sont utilisés pour préparer des systèmes à base de polyuréthane (PU) stables à l'hydrolyse, de préférence un polyuréthane thermoplastique (TPU), une mousse souple et une mousse rigide de PU, ainsi que des élastomères coulés à chaud de PU.
